# EUROPEAN PATENT SPECIFICATION

(11) **EP 1 632 179 B1**
(45) Date of publication and mention of the grant of the patent: **16.01.2008**
(21) Application number: 05018765.7
(22) Date of filing: 30.08.2005
(51) Int. Cl.: A61B 5/15

(54) **Lancet with needle protector**
Lanzette mit Nadelschutz
Lancette avec un protecteur d'aiguille

(30) Priority: 02.09.2004 JP 2004255301
(43) Date of publication of application: 08.03.2006
(73) Proprietor: NIPRO CORPORATION, Kita-ku, Osaka-shi, Osaka-fu, 531-8510 (JP)
(72) Inventor: Mori, Takeshi c/o Nipro Corporation, Osaka-shi Osaka-fu, 531-8510 (JP)
(74) Representative: Banzer, Hans-Jörg

(56) References cited:
- EP-A- 1 405 596
- EP-A- 1 430 834
- WO-A-97/39786
- US-A1- 2002 087 180
- US-A1- 2002 123 721

## Description

### BACKGROUND OF THE INVENTION

The present invention relates to a lancet for piercing a surface of a living body with a piercing needle of the lancet and extracting a required amount of body fluid such as blood or intercellular fluid in order to examine the body fluid.

In general, a test sample of body fluid such as blood or intercellular fluid is required for various medical purposes. As an important case, for example, many diabetic patients have to periodically determine glucose content in the blood so as to find out the time when insulin injection is necessary.

In order to determine a glucose content in the blood, typically, it is effective that a patient personally takes a blood sample by piercing a blood vessel through the skin, and analyzes the glucose content in the blood sample by a simplified measuring instrument. In many cases, in order to obtain a desired blood sample, a piercing set provided with a needle is used to pierce a blood vessel through a skin surface such as a finger tip, to cause a slight amount of blood to be collected.

Although various types of such piercing sets have been devised hitherto, generally, a lancet 1001 as shown in Fig. 11 (for example, see US 3358689, JP-A-06-023505) is attached to the distal end of a piercing set body 1051 as shown in Fig. 12(a) (for example, see JP-A-59-160441, JP-A-03-141929, JP-A-2000-237172, JP-A-2003-511184), and a cap 1060 shown in Fig. 12 (c) having an opening at the distal end thereof is attached for use. In the description of these drawings, the upper side of the piercing set and the lancet is referred to as the "distal end" or "front", the lower side thereof is referred to as the "rear end" or "rear", the vertical direction is referred to as the "axial direction", the side of the axis of the lancet with a needle protector is referred to as the "inside", and the outer side of the lancet referred to as the "outside".

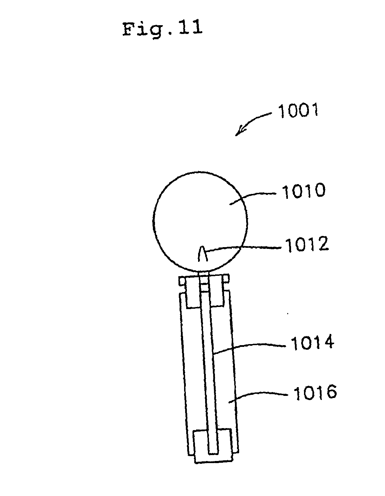

In Fig. 11, the lancet 1001 includes a lancet hub 1016 of, for example, a rectangular column-shape, with a needle 1014 having a pointed distal end and fixed in the axial direction, and a needle point protecting member 1010 arranged thereon so as to cover the needle point 1012. The needle point protecting member 1010 is fitted on the needle point 1012 to protect a user from accidental injury.

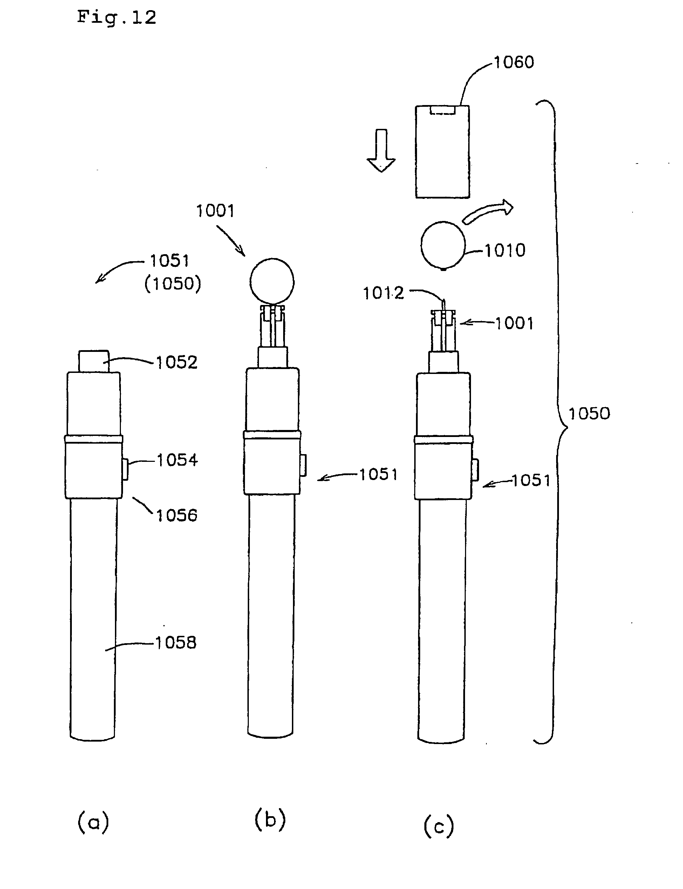

In Fig. 12(a), the piercing set body 1051 includes a cock 1058 disposed in a cylindrical casing 1056 so as to move in the axial direction, and a trigger button 1054 for releasing engagement of the cock 1058 from the engaged state provided on the side thereof. The casing 1056 includes a lancet carrier 1052 having an opening at the distal end thereof, and the lancet 1001 can be detachably held with the rear end fitted in the opening (see Fig. 12(b))

The piercing body 1051 in this structure is configured in such a manner that the cock 1058 can be retracted while compressing a coil spring, not shown, built in the piercing set body 1051 by pulling the cock 1058 toward the rear end. The retracted cock 1058 is engaged at the retracted position by an engaging mechanism, not shown, which is interlocked with the trigger button 1054. When the trigger button 1054 is pressed, the engaged state of the cock 1058 is released, and the cock 1058 is urged toward the distal end of the casing 1056 by a resilient repulsion of the compressed coil spring and hence moved forward. By the forward movement of the cock 1058, the needle point 1012 of the lancet 1001 held at the distal end of the lancet carrier 1052 slightly projects through the opening of the distal end of the cap 1060 shown in Fig. 12 (c) and is pierced into the surface of the living body. Then, the cock 1058 retracts toward the rear end using a force of the expanded coil spring to restore the natural length thereof.

The piercing set 1050 described above is prepared before piercing in such a manner that the lancet 1001 is attached to the lancet carrier 1052 of the piercing body 1051 (Fig. 12(b)), the needle point protecting member 1010 for protecting the needle point 1012 of the lancet 1001 is twisted off, and the cap 1060 of the piercing set 1050 is attached (Fig. 12(c)). After having completed piercing, the cap 1060 is removed from the piercing set 1050, and the lancet 1001 is removed from the piercing body 1051 to be discarded. In order to avoid infection via the lancet 1001 on which blood remains, the lancet 1001 is replaced for each piercing, and discarded after use.

However, in the piercing set 1050 in the related art as described above, the sharp needle point of the lancet 1001 is exposed as shown in Fig. 12 (c) when the needle point protecting member 1010 is twisted off. Therefore, when the cap 1060 of the piercing set 1050 is attached thereafter, there may be a case of injuring a fingertip or the like by accident.

There is also a similar risk when removing the cap from the piercing device after use. In addition, when the nurse or the like pierces the patient in a hospital ward, such erroneous piercing after use results in the risk of infection of HIV, hepatitis B virus and the like, and hence it involves substantial risks.

In order to avoid such risks, a piercing set in which the needle point is not exposed except during piercing has been devised.

For example, in JP-A-03-30757, a lancet including a base unit which can be reused and a disposable end cap is disclosed. The disposable end cap can accommodate the lancet therein before and after use of the lancet. Therefore, the used lancet can be discarded together with the end cap without the risk of coming into contact with the user's fingers and the like. In other words, the needle point of the lancet before use is protected by the cap and the lancet after use can be discarded together with the end cap. Therefore, safety is ensured. However, since the lancet is discarded together with the cap, the patient who needs blood sampling on a daily basis is forced to suffer from an economical burden.

In US 2002/0087180 A1, a blood lancet is described which has a base, a needle mounted to the base, a cap that slides relative to the base, and a locking member located inside between the base and the cap. The blood lancet generally corresponds to the preamble of claim 1.

In US 2002/0123721 A1, a safety-sheathed blood sampling needle is disclosed. The needle is housed in a protective hollow body and held in place with a non-tensioned elastic band.

In WO 97/39786, a safety hypodermic needle is disclosed, which has a blunt inner tube and a sharp outer tube slidable on the inner tube. A retention means, which can have the form of resilient bands, is used to either retain the other tube in a safety position or to extend the outer tube in an activated position.

In EP 1 405 596 A1, a lancet and a piercing device are described. The lancet includes a case having an internal space and a lancing unit having a lancing needle. The lancing unit is movable within the internal space from a wait position to an advanced position.

In EP 1 430 834 A2, a shieldable needle device is disclosed, which comprises a leaf-spring-driven shield. The device includes a tip guard which telescopes over a needle cannula from a retracted position to an extended position. For this purpose, the device may comprise a leaf spring.

Accordingly, after having devoted ourselves to study, the inventors devised a lancet in which a needle point is prevented from being exposed and which is less expensive and easy to handle, and reached the present invention.

In other words, it is an object of the present invention to provide a lancet with a needle protector, in which the needle is prevented from being exposed from a piercing set before and after piercing, and the risk of erroneous piercing is significantly reduced in comparison with lancets in the related art.

Another object of the present invention is to provide an easy-to-handle lancet with a needle protector, which can be manufactured at a low cost and can be used with a commercially available multipurpose piercing set.

### SUMMARY OF THE INVENTION

In view of the above objects, the present invention provides a lancet with a needle protector according to claim 1. The dependent claims define preferred and advantageous embodiments of the invention.

A lancet with a needle protector according to the present invention includes a needle having a needle point; a lancet hub for supporting the needle so that the needle point can be exposed and having a distal end portion and a rear end portion; a cylindrical needle protector slidably fitted on the distal end portion of the lancet hub and extending beyond the needle point and capable of moving in the axial direction to expose the needle point; and a resilient member connecting the needle protector and the lancet hub and biasing the needle protector toward the distal end portion ; and a needle protector supporting portion for accommodating the rear end portion of the lancet hub. Preferably, the resilient member connects the needle protector and the needle protector supporting portion and biases the needle protector toward the distal end portion. The cylindrical needle protector, the resilient member, and the needle protector supporting portion are integrally formed.

The lancet with a needle protector according to the present invention is preferably such that the resilient member is a band-shaped resilient member.

The lancet with a needle protector according to the present invention may be such that the resilient member is a coil spring.

The lancet with a needle protector according to the present invention may include a removeable needlepoint protecting member for protecting the needlepoint of the needle.

The lancet with a needle protector according to the present invention is preferably such that the needle point protecting member and the lancet hub are integrally formed.

The lancet with a needle protector according to the present invention may be such that the needlepoint protecting member and the needle protector are integrally formed.

The lancet with a needle protector according to the present invention may be such that the needle protection member includes an inner projection which covers and can protect the needle point and an outer projection which is disposed on the outer peripheral surface of the needle protector.

The lancet with a needle protector according to the present invention preferably includes engaging means formed on the side surface of the distal end portion of the lancet hub, and engaged means formed on the rear end portion of the needle protector to be engaged with the engaging means. Therefore, the movement of the needle protector and the lancet hub can be restrained after piercing.

In the lancet with a needle protector according to the present invention, the needle protector extends to a point beyond the needlepoint even in a state in which the needle point protecting member is twisted off. Therefore, since the needle protector protects the needle point except during piercing, the risk of erroneous piercing can be significantly reduced in comparison with the

### related art.

In the lancet with a needle protector according to the present invention, the lancet hub, the needlepoint protecting member and the needle can be integrally formed using a metal die. On the other hand, the needle protector, the resilient member and the needle protector supporting member can also be integrally formed using a metal die. Since the lancet with a needle protector according to the present invention can be formed by fitting these two integrally formed parts, it can be manufactured at a significantly low cost. In addition, it can also be used with commercially available multipurpose piercing sets without replacing the piercing set by purchase, which is economically advantageous.

The lancet with a needle protector according to the present invention may be configured to connect the needle protector and the needle protector supporting member by a curved resilient member. The inwardly projecting engaged means can be formed on the rear end portion of the needle protector, and the engaging means can be provided on the side surface near the distal end portion of the lancet hub. Therefore, the lancet hub is not engaged with the needle protector in the normal state. However, by pressing the resilient member from the outside toward the center axis thereof, straightening the curved resilient member and moving the needle protector connected to the resilient member toward the distal end by a predetermined distance after piercing is completed, the engaged means is engaged with the engaging means. Accordingly, from then on, the movement of the protector 2 toward the rear end is restrained, and the needle point of the needle is held in the needle protector, whereby the lancet with a needle protector of the present invention can be discarded safely, and the possibility of being reused can be reduced.

The lancet with a needle protector according to the present invention can be mounted to and dismounted from the piercing set the same as a commercially available lancet in the related art. In addition, since the needle point is not exposed like the lancet in the related art, it can be handled without anxiety.

### BRIEF DESCRIPTION OF THE DRAWINGS

Fig. 1 (a) is a plain view showing a lancet with a needle protector of the present invention and Figs. (b) and (c) are cross-sectional views thereof.
Fig. 2 (a) is a plain view showing a lancet with a needle protector, and Figs. (b) and (c) are cross-sectional views thereof.
Fig. 3 is an enlarged view of a section of a resilient member of a lancet with a needle protector according to the present invention.
Fig. 4(a) is a plain view showing an example of a general piercing set to which the lancet is mounted, and Fig. 4(b) is a cross-sectional view of the piercing set in Fig. 4(a) on which the lancet is mounted.
Figs. 5(a), (b) and (c) are explanatory drawings showing a state of preparation of the lancet with a needle protector according to the present invention before puncturing.
Figs. 6(a), (b), (c), (d), and (e) are explanatory drawings showing the movement of the lancet with a needle protector according to the present invention during piercing.
Fig. 7 is an explanatory drawing showing a state of removing the lancet with a needle protector according to the present invention from the piercing set after use.
Fig. 8(a) is a plain view showing a lancet with a needle protector according to another embodiment of the present invention and Fig. 8(b) is a vertical cross sectional view thereof.
Fig. 9 (a) is a plain view showing a lancet with a needle protector according to still another embodiment of the present invention, and Fig. 9(b) is a vertical cross-sectional view.
Fig. 10 (a) is a plain view of a lancet with a needle protector and Fig. 10(b) is a vertical cross-sectional view thereof.
Fig. 11 is a plain view showing a lancet in the related art.
Fig. 12(a) is a plain view showing a main body of a general piercing set to which a lancet is mounted, Fig.
12(b) is an appearance view of the piercing set with the lancet mounted, and Fig. 12(c) is an appearance view showing the piercing set in a state in which the needlepoint protecting member is removed from the lancet and a cap is fitted on the main body.

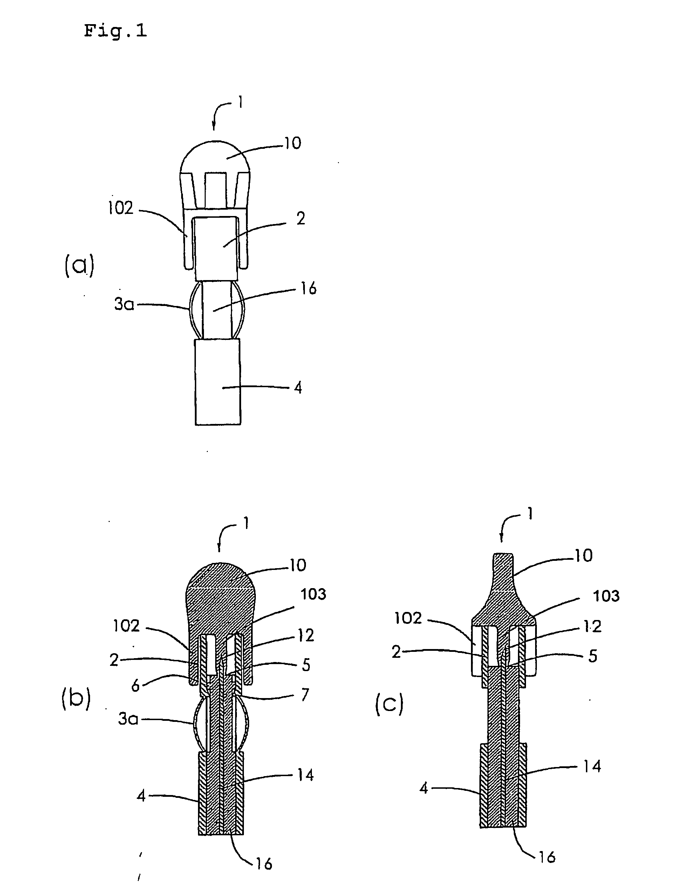

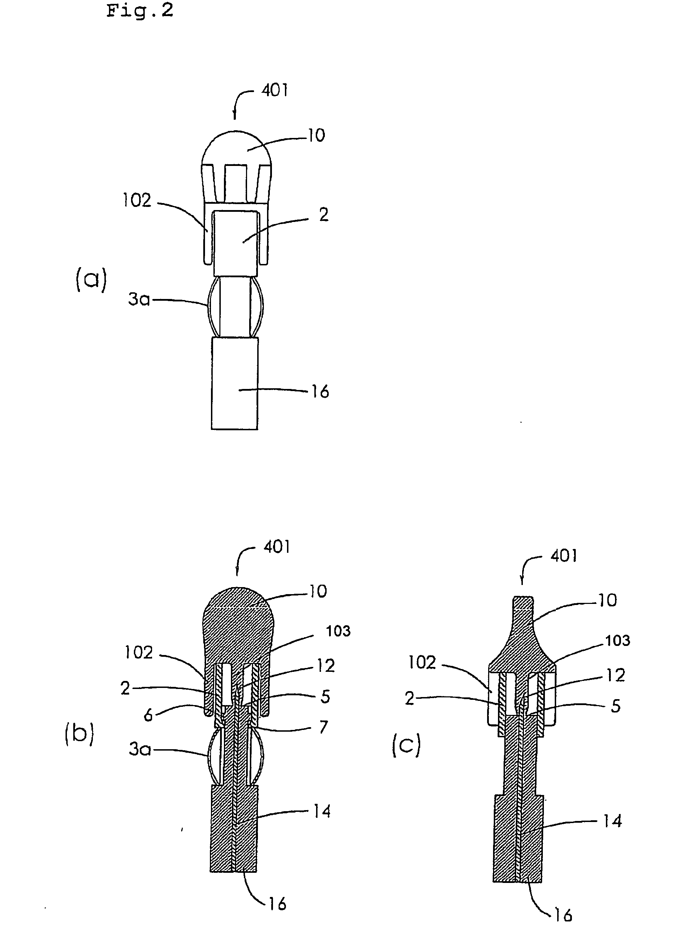

A lancet with a needle protector according to the first embodiment of the present invention includes, as shown in Fig. 1(a) and Fig. 1(b) and Fig. 1(c), which are cross-sectional views of Fig. 1(a), a needle 14, a lancet hub 16 for supporting the needle so that the needlepoint 12 can be exposed, a cylindrical needle protector 2 fitted on the distal end portion of the lancet hub 16 so as to be capable of moving in the axial direction, a needle protector supporting member 4 for accommodating the rear end portion of the lancet hub 16, a resilient member 3a for connecting the needle protector 2 and the needle protector supporting member 4. Alternatively, as a lancet 401 with a needle protector according to an example shown in Fig. 2(a), and Fig. 2(b) and Fig. 2(c), which are cross-sectional views of Fig. 2(a), it is possible to eliminate the needle protector supporting member 4 and connect the needle protector 2 and the lancet hub 16 with the resilient member 3a. The lancet 1 with a needle protector of a structure as shown in Fig. 1 will be described below. As noted above, in the description of the drawings, the upper side is represented as "distal end" or "front", the lower side is referred to as the "rear end" or "rear", the vertical direction is referred to as the "axial direction", the side of the axis of the lancet with a needle protector is referred to as the "inside", and the outer side is referred to as the "outside" or "out" in description.

The lancet hub 16 includes the needle 14 fixed to the center axis thereof, and the needlepoint protecting member 10 covered on the distal end of the needle to protect the needle point 12. Although the needle protecting member 10 may be a detachably attached cap separate from the lancet hub 16, it is preferably formed integrally with the lancet hub 16. When forming integrally, a thin breakable portion 5 is formed between the needlepoint protecting member 10 and the lancet hub 16, and the needlepoint protecting member 10 can be separated from the lancet hub 16 by breaking the breakable portion 5 by, for example, twisting it.

The needle protector 2 is formed with an inwardly projecting engaged means 7 on the rear end portion of the needle protector 2, which can be engaged with an engaging means 6 projecting outward from the side surface near the distal end portion of the lancet hub 16.

The curved resilient member 3a as shown in Fig. 1(a) is preferably flexible so as to minimize the reduction of speed of piercing during piercing, and on the other hand, it needs to have a strength which can hold the needle protector 2. Therefore, the resilient member 3a is preferably formed of thermoplastic resin such as polypropylene, polyethylene, polystyrene or polyacetal. It is in terms of cost and assembling efficiency to integrally form the needle protector 2, the resilient member 3a, and the needle protector supporting member 4.

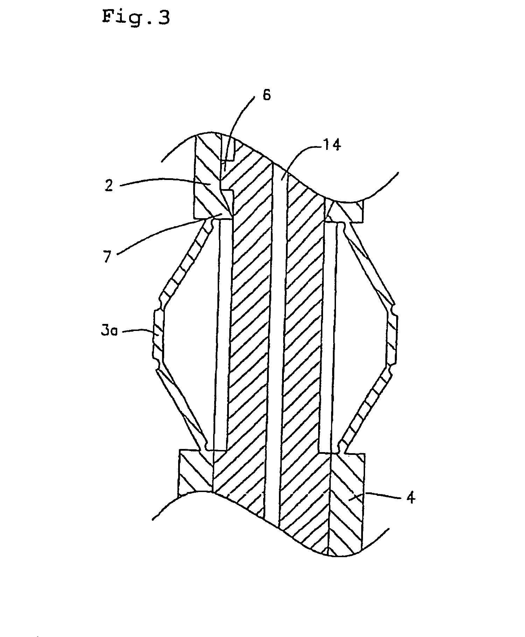

In the present embodiment, the shape of the resilient member 3a is not specifically limited, and as shown in Figs. 1(a) and (b), it may be an elongated band-shaped leaf spring. The resilient member 3a needs not have an arcuate shape, and may have, for example, a polygonal shape. Alternatively, in order to provide flexibility and strength together, the resilient member 3a may be partly provided with a thin flexible portion as shown in Fig. 3. The resilient member 3a is connected at the distal end portion with the needle protector 2 so that the needle protector 2 is movably fitted on the distal end portion of the lancet hub 16. However, the rear end portion may be directly connected to the lancet hub 16 as with the lancet 401 with the needle protector, or may be connected to the needle protector supporting member 4 which accommodates and fixes the rear end portion of the lancet hub 16 as with the lancet 1 with a needle protector.

As described above, in the lancet 1 with a needle protector according to the present invention, the needle protector supporting member 4 which is connected to the needle protector 2 via the resilient member 3a accommodates and fixes the lancet hub 16. The shape of the bottom surface of the needle protector supporting member 4 is not specifically limited, and may be a circular or polygonal shape, as long as it has a size and a shape which can be fixed to a lancet carrier of a commercially available piercing set.

The length and the resiliency of the resilient member 3a are set to an extent that the needle point 12 is exposed when the needle protector 2 moves to the rear most end and the needle protector 2 does not come apart from the lancet hub 16 at the distal end side when the protector 2 is moved to the distal most end.

The lancet 1 with a needle protector according to the present invention configured as described above is adapted in such a manner that the needle protector 2 embraces the needle 14 to a point beyond the needle point 12, and the needle protector 2 protects the needle point 12 except during piercing even when the needle point protecting member 10 is twisted off, and hence the risk of erroneous piercing can be significantly reduced in comparison with the related art.

Subsequently, a preferred method of manufacturing the lancet 1 with a needle protector according to the first embodiment of the present invention will be described.

As described above, it is preferable to mold the lancet hub 16, the needle point protecting member 10 and the needle 14 integrally by forming a metal mold into the shape of a contour of these members in advance. In a first step, the needle 14 formed of metal such as stainless steel, or synthetic resin is fixed in the axial direction at the center of the mold. In this case, the needle 14 is fixed with the needle point 12 oriented toward the distal end. Subsequently, thermoplastic resin such as polyethylene or polypropylene, which is in the fluid state at a high temperature, is filled into the mold. When the content of the mold is sufficiently cured by cooling, the metal mold is removed to take out the integrally molded lancet hub 16, the needle 14, and the needlepoint protecting member 10. Although the molding material of the lancet hub 16 and the needlepoint protecting member 10 is preferably thermoplastic resin, it is not specifically limited.

Similarly, the needle protector 2, the resilient member 3a, and the needle protector supporting member 4 are also formed integrally with an already prepared metal die. The molding materials are the same as those described above.

Subsequently, the rear end side of the integrally formed three components, i.e., lancet hub 16, the needle 14, and the needlepoint protecting member 10 is inserted from the distal end side of the integrally formed three components, i.e., needle protector 2, resilient member 3a and the needle protector supporting member 4. While the engaging means 6 formed on the side surface of the distal end of the lancet hub 16 is not engaged with the inwardly projecting engaged means 7 at the rear end portion of the needle protector 2, the engaging means 6 is inserted to a position where the needle point 12 of the needle 14 is accommodated in the needle protector 2. Then, the rear end portion of the lancet hub 16 is fixed to the needle protector supporting member 4, whereby the lancet 1 with a needle protector according to the first embodiment of the present invention is completed.

As described above, the lancet 1 with a needle protector of the present invention as described above can be formed by molding the lancet hub 16, the needle point protecting member 10, and the needle 14 integrally by using a metal mold while molding the needle protector 2, the resilient member 3a and the needle protector supporting member 4 integrally by using a metal mold, and fitting both of them together. Therefore, the lancet 1 with a needle protector according to the present invention can be manufacture at relatively low cost. Also, it is economically advantageous because it can be used together with a commercially available multipurpose piercing set without replacing the piercing set bodies 51, 1051 by purchase.

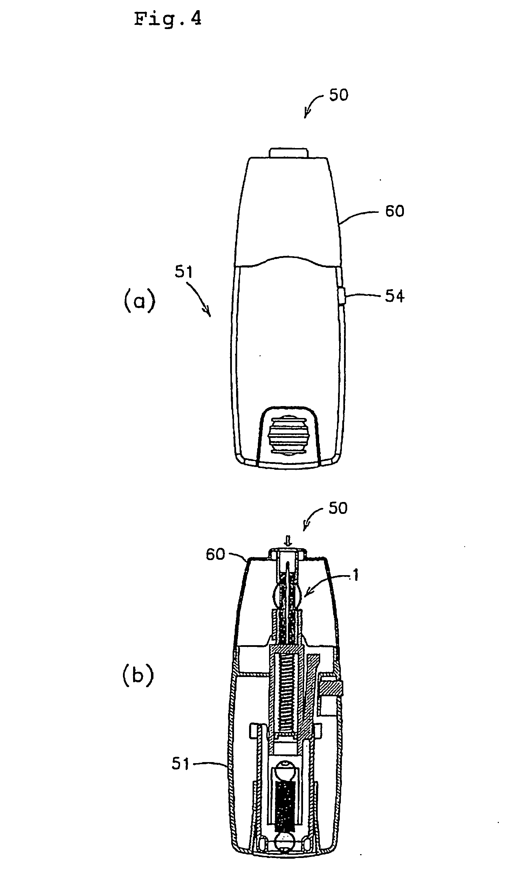

Hereinafter, an operation of the lancet 1 with a needle protector when the lancet 1 with a needle protector according to this embodiment is mounted to the piercing set 50 shown in Figs. 4(a), (b) for piercing will be described. Fig. 4(b) is a cross sectional view of the lancet 1 with a needle protector of the present invention mounted to the piercing set 50 shown in Fig. 4(a). Since the piercing set 50 is a known piercing set which is commercially available like the piercing set 1050, the structure and the function thereof will be described only briefly.

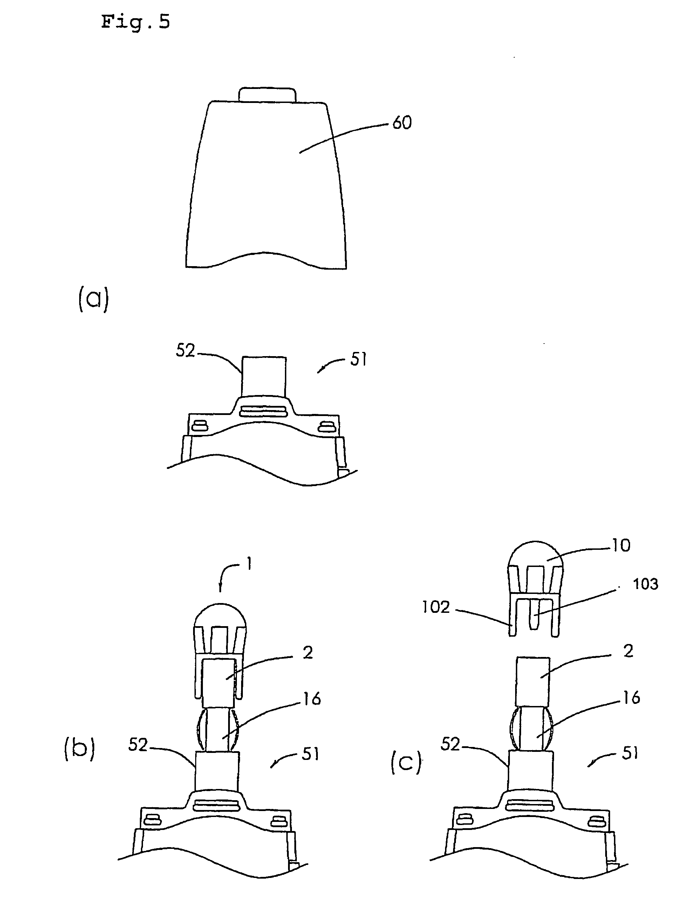

As preparation for piercing, a cap 60 is removed from the piercing set 50 as shown in Fig. 5(a), and the lancet 1 is mounted to a lancet carrier 52 as shown in Fig. 5(b). Then, as shown in Fig. 5(c), the needle point protecting member 10 is twisted off from the lancet 1.

Although the needlepoint protecting member 10 is integrally formed with the lancet hub 16 as described above, since the breakable portion 5 is thinned, it is broken by twisting off the needle protecting member 10, and is separated from the lancet hub 16. At this time, since the needlepoint 12 of the needle 14 is embraced by the needle protector 2, as will be seen when compared with Figs. 12(a) to 12(c) which show the procedure of preparation in the related art, the needlepoint 12 is prevented from being exposed, and hence there is no risk of erroneous piercing.

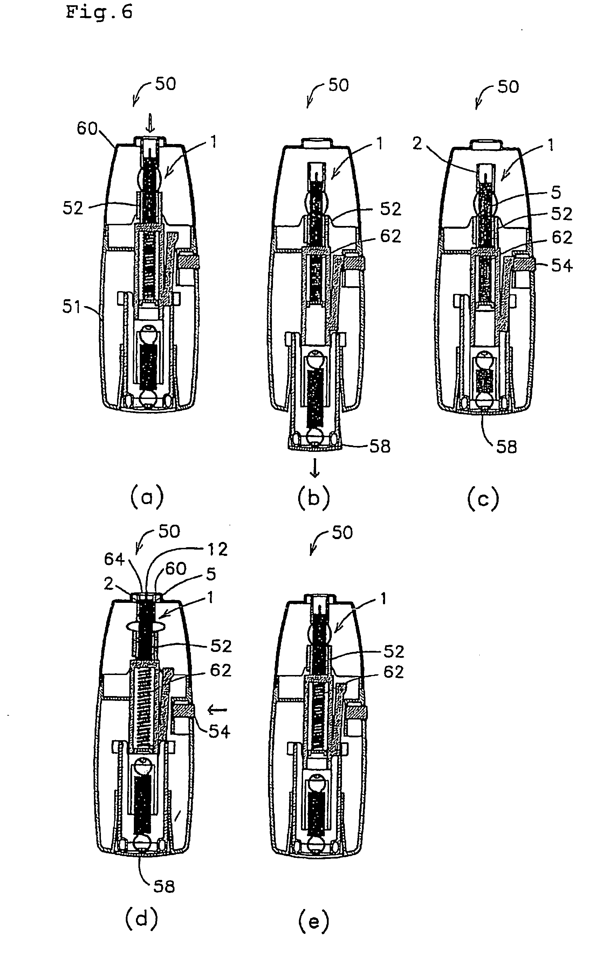

Subsequently, referring to Figs. 6(a) to Fig. 6(c), the state during piercing will be described.

As shown in Fig. 6(a), the piercing set body 51 is mounted to the cap 60.

As shown in Fig. 6(b), when the cock 58 is pulled and the lancet-launching spring 62 in the piercing set 50 is compressed, the lancet carrier 52 on which the lancet 1 is mounted is retracted, thereby getting into the ready-to-piercing state (engaged state) as shown in Fig. 6(c).

Then, when the trigger button 54 is pressed as shown in Fig. 6(d), compression of the lancet-launching spring 62 is released, and the lancet 1 rushes toward the distal end with the lancet carrier 52. At this time, the forward movement of the needle protector 2 is stopped by coming into abutment with the inner surface of the cap 60. However, since the lancet hub 5 can move within the needle protector 2, it keeps moving forward toward the distal end by inertia after the forward movement of the needle protector 2 is stopped, and the needle point 12 of the needle 14 ejects out from the distal opening 64. Piercing into the surface of the living body is achieved at this moment.

Subsequently, the lancet carrier 52 returns to the initial position (Fig. 6(a)) together with the lancet 1 as shown in Fig. 6(e) when the lancet-launching spring 62 is restored to its natural length.

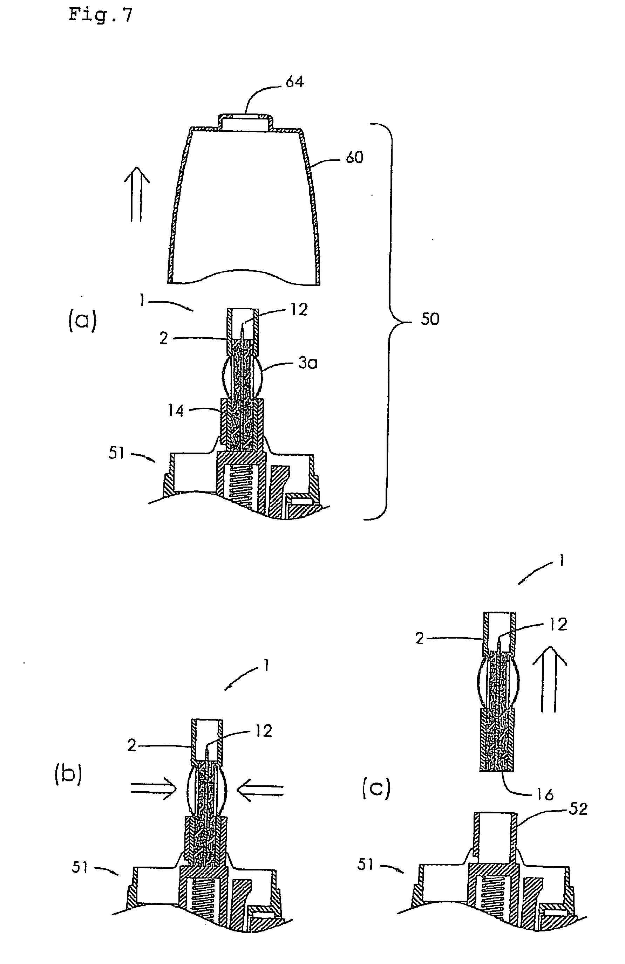

Removal of the lancet 1 is achieved by removing the cap 60 from the piercing set 50 as shown in Fig. 7(a) first. At this time as well, since the needle point 12 is protected by the needle protector 2, the needlepoint 12 is prevented from being exposed, and hence there is no risk of accidental piercing.

Subsequently, the resilient member 3a is pressed from the outside toward the center axis as shown in Fig. 7(b), and the curved resilient body 3a is straightened to move the needle protector 2 toward the distal end of the lancet 1. Since the engaged means 7 is engaged with the engaging means 6 when the needle protector 2 moves in the axial direction by a predetermined distance, movement of the needle protector 2 toward the rear end will be restrained. Therefore, the needlepoint 12 of the needle 14 is retained within the needle protector 2, and thence the lancet 1 can be discarded safely and is prevented from being easily reused .

As described above, the lancet 1 with a needle protector according to the present invention is easy to mount to and dismount from the piercing set 50 similar to a commercially available lancet in the related art. Even when the needlepoint protecting member 10 is twisted off, the needle protector supporting member 4 can embrace the space beyond the needle point 12. Since the needle point 12 is protected by the needle protector 2 other than at the time of piercing, the risk of accidental piercing can be reduced significantly in comparison with a lancet in the related art.

The lancet 1 with a needle protector according to the present invention can be formed by molding the lancet hub 16, the needlepoint protecting member 10 and the needle 12 integrally using a metal mold. On the other hand, the needle protector 2, the resilient member 3a and the needle protector supporting member 4 can also be formed integrally using a metal mold. Since the lancet 1 with the needle protector of the present invention can be formed by fitting these integrally formed two parts, it can be manufactured at a relatively low cost. It is also economically advantageous since it can be used with a commercially available multipurpose piercing set without replacing the piercing set by purchase.

Furthermore, the lancet 1 with a needle protector according to the present invention is configured in such a manner that the needle protector 2 and the needle protector supporting member 4 are connected with the curved resilient member 3a as shown in Fig. 1. Since the inwardly projecting engaged means 7 is formed on the rear end portion of the needle protector 2, and the engaging means 6 is provided on the side surface near the distal end portion of the lancet hub, the engaged means 7 is engaged with the engaging means 6 of the lancet hub 16 when the resilient body 3a is pressed toward the center axis and straightened to move the needle protector 2 connected to the resilient member 3a toward the distal end of the lancet 1 by a predetermined distance after piercing is completed. Therefore, movement of the needle protector 2 toward the rear end from then on is restrained, and hence the needlepoint 12 of the needle 14 is held within the needle protector 2, whereby the lancet 1 with the needle protector of the present invention can be discarded safely and prevented from being easily reused.

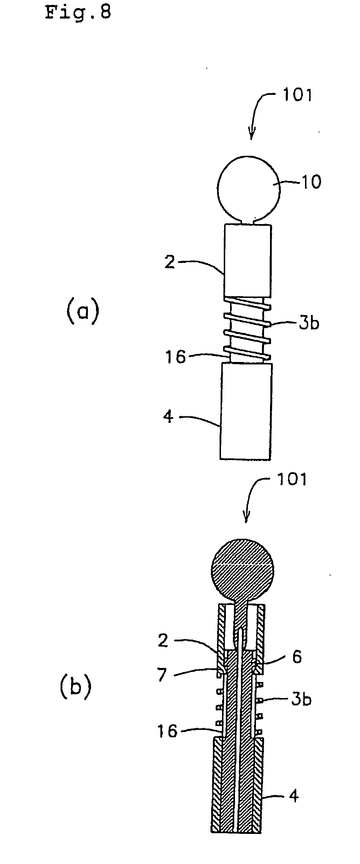

Although one embodiment of the present invention has been described thus far, the lancet with a needle protector according to the present invention is not limited thereto. Although, in the lancet 1 with the needle protector of the above-described embodiment, the resilient member is the curved resilient member, it may be a coil spring 3b which is interposed between the needle protector 2 and the needle protector supporting portion 4 and turns around the lancet hub 16 as shown in Figs. 8(a) and (b). Other structures, functions, manufacturing method, materials, and the like may be the same as the lancet 1 with a needle protector in the above-described embodiment. In the following description, the same components as the lancet 1 with a needle protector in the above-described embodiment are represented by the same reference numerals.

Therefore, since the lancet 101 with a needle protector of the present invention is configured in such a manner that the needle protector 2 protects the needle point 12 except during piercing in the same manner as the lancet 1 with a needle protector, the risk of erroneous piercing can be reduced significantly in comparison with the related art, and the lancet is easy to handle and safe.

The lancet 101 with a needle protector according to the present invention can be formed by fitting two integrally formed parts, it can be manufactured at relatively low cost, and since it can be mounted to a commercially available piercing set in the related art, it is economically advantageous.

Since the lancet 101 with a needle protector is provided with the engaging means 6 and the engaged means 7 to restrain the movement of the lancet hub 16, it can be safely discarded after piercing and prevented from being easily reused. Different from the lancet 1 with a needle protector described above, the lancet 101 with a needle protector is configured to move the needle protector 2 toward the distal end of the lancet hub 16 by directly holding the needle protector 2 after having used the lancet 101, and to engage the engaged means 7 and the engaging means 6 before discarded.

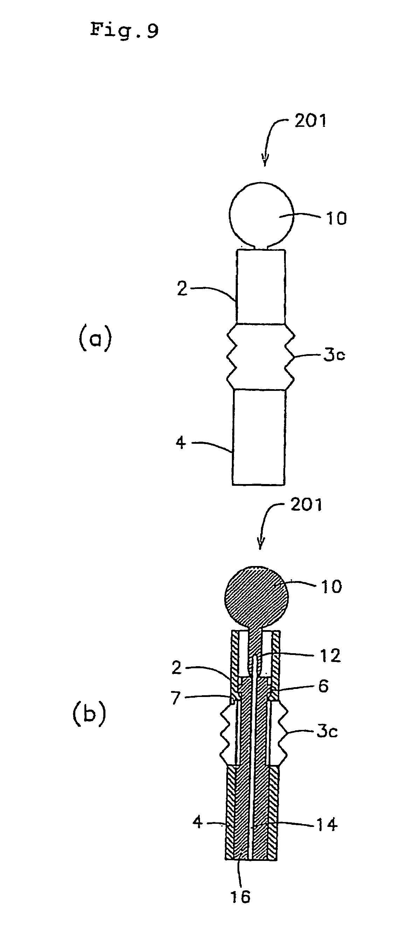

The lancet with a needle protector according to the present invention may also include a resilient member 3c formed into an accordion shape as shown in Figs. 9(a) and 9(b). Other structures, functions, manufacturing method, materials, and the like of a lancet 201 with a needle protector may be the same as the same as the lancet 1 with a needle protector in the above-described embodiment.

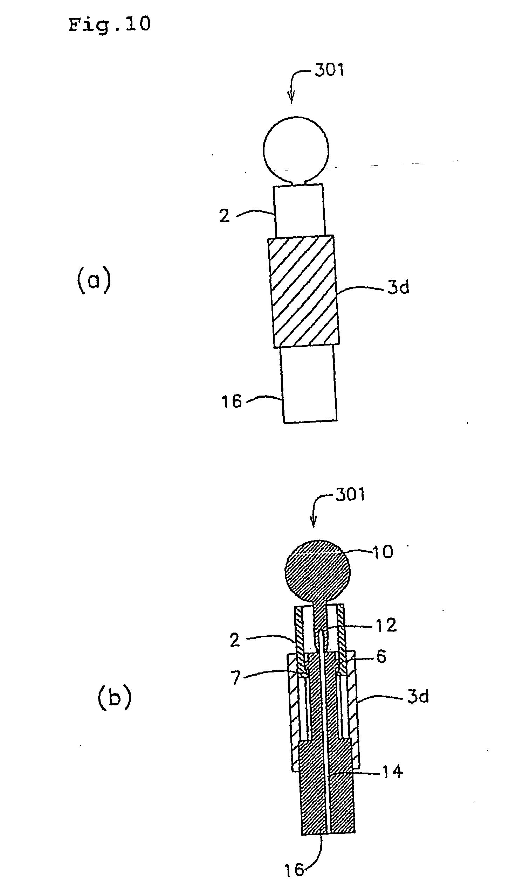

Therefore, in the same manner as the above-described lancet 1 with a needle protector, the lancet 201 with a needle protector in this embodiment is also configured in such a manner that the needle protector 2 protects the needle point except during piercing to reduce the risk of accidental piercing significantly after piercing in comparison with the related art, and hence it can be handled easily and safely. Since the lancet 201 with a needle protector can be formed by fitting two integrally formed parts, it can be manufactured at relatively low cost, and since it can be mounted to a commercially available piercing set in the related art, it is economically advantageous. In addition, since the lancet 201 with a needle protector is provided with the engaging means 6 and the engaged means 7 to restrain the movement of the lancet hub 16, the needle protector 2 can be slid by pressing the projections of the accordion from the outside and the engaged means 7 and the engaging means 6 can be engaged after use. Therefore, the lancet 201 with a needle protector according to the present embodiment can be discarded safely after piercing like those in the above-described embodiment, and may be prevented from being easily reused. Alternatively, a lancet with a needle protector may include a resilient member formed of an expandable cylindrical rubber 3d as shown in Figs. 10 (a) and 10 (b). The lancet 301 with a needle protector is formed by integrally molding the lancet hub 16, the needlepoint protecting member 10 and the needle 14, and is covered by the needle protector 2 formed separately. Subsequently, the rubber 3d is covered on the lancet hub 16 and the needle protector 2 to connect therebetween for piercing. Other structures, function, manufacturing method, material, and the like may be the same as the lancet 1 with a protector in the above-described embodiment.

In the same manner as the above-described lancet 1 with a needle protector, the lancet 301 with a needle protector in this example is also configured in such a manner that the needle protector 2 protects the needle point 12 except during piercing to reduce the risk of accidental piercing significantly after piercing in comparison with the lancet in the related art, and hence it can be handled easily and safely. The lancet 301 with a needle protector can be manufactured relatively easily, and since it can be mounted to a commercially available piercing set in the related art, it is economically advantageous.

In addition, the lancet 301 with a needle protector is provided with the engaging means 6 and the engaged means 7 to restrain the movement of the lancet hub 16, and the needle protector 2 can be slid by expanding the rubber 3d, and the engaged means 7 and the engaging means 6 can be engaged after use. Therefore, the lancet 301 with a needle protector according to this example can be discarded safely after piercing as in the above-described embodiments and can be prevented from being reused easily.

As described above, in the lancet with a needle protector of the present invention, the shape of the resilient member 3 is not specifically limited as long as it has a size and shape which can be accommodated in the cap of the piercing set such as an elongated band-shape leaf spring, a helical coil spring, and an accordion shape. It is also possible to reduce the thickness partly to provide a flexible portion. The resilient member 3 is preferably formed by molding thermoplastic resin such as polypropylene, polyethylene, polystyrene, and polyacetal. However, the material is not specifically limited as long as it can be expanded freely.

The needlepoint protecting member 10 may be any type as long as it protects the needlepoint 12 and can be held for breaking the breakable portion 5 as shown in Figs. 8 to 10. However, it may be provided with an inward projection 103 which can protect the needle point 12 and an outward projection 102 which can be disposed on the outer peripheral surface of the needle protector 2 as shown in Fig. 1 and Fig. 2. The outward projection 102 is disposed on the outer peripheral surface of the needle protector 2 in contact, or out of contact, without pressing the needle protector 2 toward the proximal side. The outward projection 102 may be disposed over the entire circumference of the needle protector 2, but it may be provided one or more on a part of the outer peripheral surface as shown in Fig. 1 and Fig. 2. The outwardly projection 102 can prevent the needle protector 2 from moving toward the rear side with an unexpected external force before use of the lancet 1 and hence becoming difficult to be mounted to the puncture set 50 or 1050.

Although it is economically advantageous to integrally mold the needle 14 and the lancet hub 16 of the needlepoint protecting member 10, the manufacturing method is arbitrary. The needlepoint protecting member 10 and the needle protector 2 can be molded integrally, and may be manufactured separately and assembled. Materials of other members other than the resilient member are also not limited.

The size and weight of the lancet with the needle protector according to the invention can be changed as needed in accordance with the piercing set to be mounted, and are not specifically limited.

The present invention may be implemented in variously improved, changed, or modified modes based on the knowledge of those skilled in the art without departing from the scope thereof.

## Claims

1. A lancet with a needle protector comprising:
a needle (14) having a needle point (12);
a lancet hub (16) for supporting the needle (14) so that the needle point (12) can be exposed and having a distal end portion and a rear end portion;
a cylindrical needle protector (2) slidably fitted on the distal end portion of the lancet hub (16) and extending beyond the needle point (12) and capable of moving in an axial direction to expose the needle point (12);
a resilient member (3a, 3b, 3c) connecting the cylindrical needle protector (2) and the lancet hub (16) and biasing the cylindrical needle protector (2) toward the distal end portion; and
a needle protector supporting portion (4) for accommodating the rear end portion of the lancet hub (16),
**characterized in that**
the cylindrical needle protector (2), the resilient member (3a, 3b, 3c), and the needle protector supporting portion (4) are integrally formed.

2. The lancet with a needle protector according to claim 1, wherein the resilient member (3a) is a band-shaped resilient member.

3. The lancet with a needle protector according to claim 1, wherein the resilient member (3b) is a coil spring.

4. The lancet with a needle protector according to any one of the preceding claims, further comprising a removeable needlepoint protecting member (10) covering the cylindrical needle protector (2) for protecting the needlepoint (12) of the needle (14) prior to use.

5. The lancet with a needle protector according to claim 4, wherein the needlepoint protecting member (10) and the lancet hub (16) are integrally formed.

6. The lancet with a needle protector according to claim 4 or claim 5, wherein the needlepoint protecting member (10) and the cylindrical needle protector (2) are integrally formed .

7. The lancet with a needle protector according to any one of claims 4 to 6, wherein the needlepoint protecting member (10) comprises an inner projection (103) which extends over the needlepoint (12), and an outer projection (102) disposed on an outer peripheral surface of the cylindrical needle protector (2).

8. The lancet with a needle protector according to any one of the preceding claims, further comprising:
engaging means (6) formed on a side surface of the distal end portion of the lancet hub (16); and
engaged means (7) formed on a rear end portion of the needle protector (2) which can engage with the engaging means (6).

## Patentansprüche

1. Lanzette mit einem Nadelschutz, umfassend:
eine Nadel (14) mit einer Nadelspitze (12);
einen Lanzettensitz (16) zum Halten der Nadel (14), so dass die Nadelspitze (12) freigelegt werden kann, mit einem Distalendabschnitt und einem Hinterendabschnitt;
einen zylindrischen Nadelschutz (2), welcher verschiebbar auf den Distalendabschnitt des Lanzettensitzes (16) gepasst ist und sich über die Nadelspitze (12) hinaus erstreckt und in der Lage ist, sich in einer axialen Richtung zu bewegen, um die Nadelspitze (12) freizulegen;
ein elastisches Teil (3a, 3b, 3c), welches den zylindrischen Nadelschutz (2) und den Lanzettensitz (16) verbindet und den zylindrischen Nadelschutz (2) in Richtung des Distalendabschnitts vorspannt; und
einen Nadelschutzhalteabschnitt (4) zum Aufnehmen des Hinterendabschnitts des Lanzettensitzes (16),
**dadurch gekennzeichnet,**
**dass** der zylindrische Nadelschutz (2), das elastische Teil (3a, 3b, 3c) und der Nadelschutzhalteabschnitt (4) integral ausgebildet sind.

2. Lanzette mit einem Nadelschutz nach Anspruch 1, wobei das elastische Teil (3a) ein bandförmiges elastisches Teil ist.

3. Lanzette mit einem Nadelschutz nach Anspruch 1, wobei das elastische Teil (3b) eine Spiralfeder ist.

4. Lanzette mit einem Nadelschutz nach einem der vorhergehenden Ansprüche, darüber hinaus umfassend ein entfernbares Nadelspitzenschutzteil (10), welches den zylindrischen Nadelschutz (2) bedeckt, um die Nadelspitze (12) der Nadel (14) vor der Verwendung zu schützen.

5. Lanzette mit einem Nadelschutz nach Anspruch 4, wobei das Nadelspitzenschutzteil. (10) und der Lanzettensitz (16) integral ausgebildet sind.

6. Lanzette mit einem Nadelschutz nach Anspruch 4 oder Anspruch 5, wobei das Nadelspitzenschutzteil (10) und der zylindrische Nadelschutz (2) integral ausgebildet sind.

7. Lanzette mit einem Nadelschutz nach einem der Ansprüche 4-6, wobei das Nadelspitzenschutzteil (10) einen Innenvorsprung (103), welcher sich über die Nadelspitze (12) erstreckt, und einen Außenvorsprung (102), welcher an einer äußeren Umfangsfläche des zylindrischen Nadelschutzes (2) angeordnet ist, umfasst.

8. Lanzette mit einem Nadelschutz nach einem der vorhergehenden Ansprüche, darüber hinaus umfassend:
Eingriffmittel (6), welche an einer Seitenfläche des Distalendabschnitts des Lanzettensitzes (16) ausgebildet sind; und
Einrastmittel (7), welche an einem Hinterendabschnitt des Nadelschutzes (2) ausgebildet sind und welche mit den Eingriffsmitteln (6) in Eingriff kommen können.

## Revendications

1. Lancette équipée d'un protecteur d'aiguille comprenant :
une aiguille (14) possédant une pointe d'aiguille (12) ;
un embout de lancette (16) afin de supporter l'aiguille (14) de sorte que la pointe d'aiguille (12) peut être exposée et possédant une partie terminale distale et une partie terminale arrière ;
un protecteur d'aiguille cylindrique (2) fixé de manière coulissante sur la partie terminale distale de l'embout de lancette (16) et s'étendant au-delà de la pointe d'aiguille (12) et capable de se déplacer dans une direction axiale afin d'exposer la pointe d'aiguille (12) ;
un élément élastique (3a, 3b, 3c) reliant le protecteur d'aiguille cylindrique (2) et l'embout de lancette (16) et contraignant le protecteur d'aiguille cylindrique (2) vers la partie terminale distale ; et
une partie supportant le protecteur d'aiguille (4) afin d'adapter la partie terminale arrière de l'embout de lancette (16),
**caractérisée en ce que**
le protecteur d'aiguille cylindrique (2), l'élément élastique (3a, 3b, 3c), et la partie supportant le protecteur d'aiguille (4) sont formés de manière solidaire.

2. Lancette équipée d'un protecteur d'aiguille selon la revendication 1, dans laquelle l'élément élastique (3a) est un élément élastique en forme de bande.

3. Lancette équipée d'un protecteur d'aiguille selon la revendication 1, dans laquelle l'élément élastique (3b) est un ressort à enroulement.

4. Lancette équipée d'un protecteur d'aiguille selon l'une quelconque des revendications précédentes, comprenant en outre un élément protégeant la pointe d'aiguille amovible (10) recouvrant le protecteur d'aiguille cylindrique (2) afin de protéger la pointe d'aiguille (12) de l'aiguille (14) avant l'utilisation.

5. Lancette équipée d'un protecteur d'aiguille selon la revendication 4, dans laquelle l'élément protégeant la pointe d'aiguille (10) et l'embout de lancette (16) sont formés de manière solidaire.

6. Lancette équipée d'un protecteur d'aiguille selon la revendication 4 ou la revendication 5, dans laquelle l'élément protégeant la pointe d'aiguille (10) et le protecteur d'aiguille cylindrique (2) sont formés de manière solidaire.

7. Lancette équipée d'un protecteur d'aiguille selon l'une quelconque des revendications 4 à 6, dans laquelle l'élément protégeant la pointe d'aiguille (10) comprend une partie saillante interne (103) qui s'étend au-dessus de la pointe d'aiguille (12), et une partie saillante externe (102) disposée sur une surface périphérique externe du protecteur d'aiguille cylindrique (2).

8. Lancette équipée d'un protecteur d'aiguille selon l'une quelconque des revendications précédentes, comprenant en outre :
des moyens d'engagement (6) formés sur une surface latérale de la partie terminale distale de l'embout de lancette (16) ; et
des moyens d'engagement (7) formés sur une partie terminale arrière du protecteur d'aiguille (2) qui peuvent s'engager avec les moyens d'engagement (6).
